# EUROPEAN PATENT SPECIFICATION

(11) **EP 1 914 567 B1**
(45) Date of publication and mention of the grant of the patent: **01.12.2010**
(21) Application number: 07019444.4
(22) Date of filing: 04.10.2007
(51) Int. Cl.: G01S 15/89

(54) **Apparatus and method for forming an ultrasound image**
Vorrichtung und Verfahren zum Erzeugen eines Ultraschallbildes
Appareil et procédé de formation d'une image ultrasonore

(30) Priority: 17.10.2006 KR 20060100903
(43) Date of publication of application: 23.04.2008
(73) Proprietor: MEDISON CO., LTD., Kangwon-do 250-870 (KR)
(72) Inventor: Nam, Sang Gyu, 1003 Daechi-dong Gangnam-gu Seoul 135-280 (KR)
(74) Representative: Schmid, Wolfgang

(56) References cited:
- WO-A-02/16963
- WO-A-2005/050571
- JP-A- 8 322 840
- US-A- 5 390 674
- US-A1- 2004 054 284

## Description

### BACKGROUND

### 1. Field

The present invention generally relates to an ultrasound system, and more particularly to an ultrasound system for forming an ultrasound image.

### 2. Background

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional diagnostic images of internal features of an object (e.g., human organs).

Generally, in order to transmit and receive wideband ultrasound signals, the ultrasonic system comprises a plurality of transducers. When electrically stimulating a transducer, an ultrasound signal is generated and then transmitted to an object. The ultrasound echo signals reflected from the object and transmitted to the transducer are electrically converted. The converted electrical signals are amplified and signal-processed to produce image data.

The ultrasound system needs to increase the density of scan lines (i.e., number of scan lines) in forming one frame to improve the resolution of an ultrasound image. However, there is a drawback in that the frame-rate is lowered as the density of scan lines increases. Therefore, there is a need for an ultrasound system, which is capable of improving the resolution of an ultrasound image without lowering the frame-rate.

An ultrasound system for forming an ultrasound image according to the preamble of claim 1 and a method for forming an ultrasound image according to the preamble of claim 6 are known from US 2004/0054284 A1. WO 02/16963 A2 describes a method and an apparatus which produce spatially compounded panoramic ultrasonic images by electronically steering beams in a plurality of different look directions as a transducer is moved in relation to a panoramic image filed. The received echo information is compounded, then aligned and combined with previously acquired echo information to form a spatially compounded panoramic image.

### BRIEF DESCRIPTION OF THE DRAWINGS

Arrangements and embodiments may be described in detail with reference to the following drawings in which like reference numerals refer to like elements and wherein:

FIG. 1 is a block diagram illustrating one embodiment of the present invention;

FIGS. 2 to 4 are exemplary diagrams showing a group of scan lines and a frame in accordance with embodiments of the present invention;

FIG. 5 is an exemplary diagram showing a spatial compound of the respective groups of scan lines in accordance with one embodiment of the present invention.

### DETAILED DESCRIPTION OF THE PRESENT INVENTION

A detailed description may be provided with reference to the accompanying drawings. One of ordinary skill in the art may realize that the following description is illustrative only. Other embodiments of the present invention may readily suggest themselves to such skilled persons having the benefit of this disclosure.

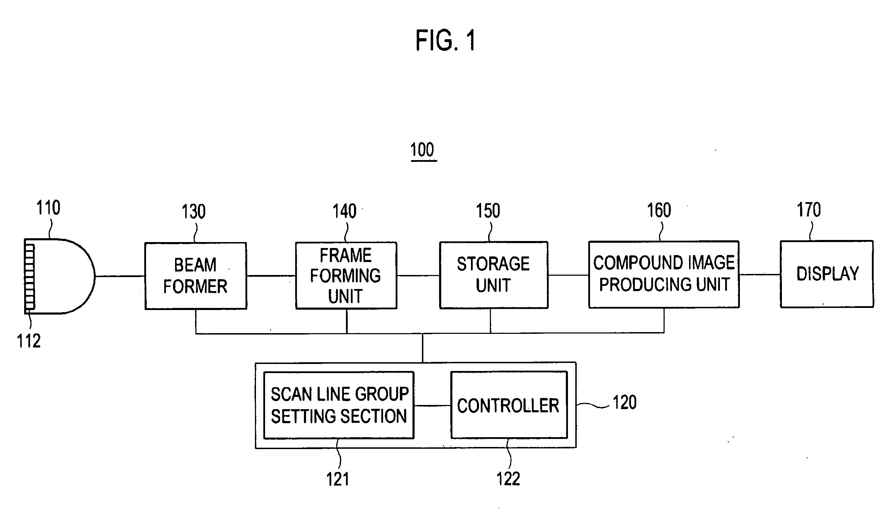

FIG. 1 is a block diagram showing one embodiment of the present invention. As shown in FIG. 1, the ultrasound system 100 includes a probe 110, a scan line setting unit 120, a beam former 130, a frame forming unit 140, a storage unit 150, a compound image producing unit 160 and a display 170. The frame forming unit 140 and the compound image producing unit 160 can be embodied as one processor. The probe 110 includes an array transducer 112 including a plurality of transducers. The probe 110 transmits and receives ultrasonic signals along the scan lines, which change at every frame change so as to form a plurality of frames for spatial compound.

The scan line setting unit 120 includes a scan line group setting section 121 and a controller 122. The scan line group setting section 121 includes a plurality of scan lines each having a predetermined reference scan line density. Based on a reference scan line group for forming a reference frame, the scan line group setting section 121 sets a group ("first scan line group") having a lower scan line density than the reference scan line density and a group ("second scan line group") having a higher scan line density than the reference scan line density.

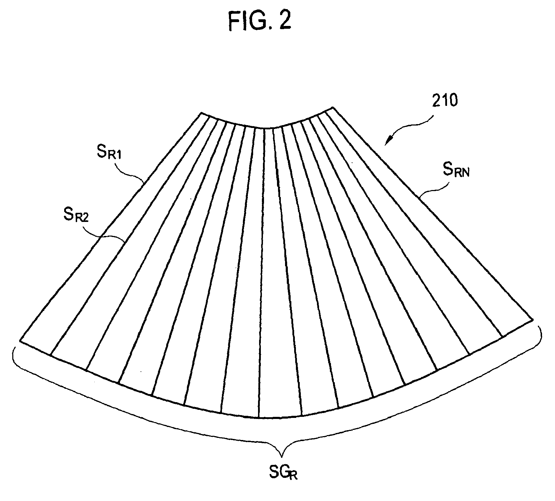

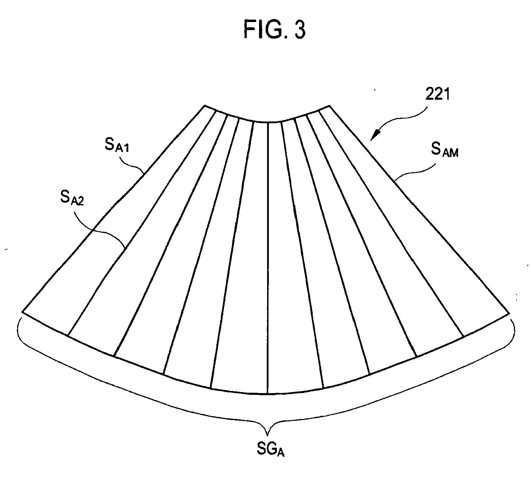

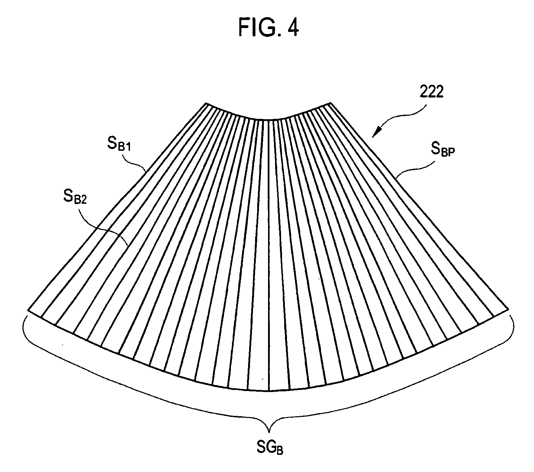

In accordance with one embodiment of the present invention, the scan line group setting section 121 sets the first scan line group (SG_{A}) and the second scan line group (SG_{B}) based on the reference scan line group (SG_{R}). As shown in FIG. 2, the reference scan line group (SG_{R}) includes a plurality of scan lines (S_{R1} to S_{RN}) having a predetermined reference scan line density. The first scan line group (SG_{A}) includes a plurality of scan lines (S_{A1} to S_{AM}) each having a lower scan line density than the reference scan line density as shown in FIG. 3. Further, the second scan line group (SG_{B}) includes a plurality of scan lines (S_{B1} to S_{BP}) each having a higher scan line density than the reference scan line density as shown in FIG. 4. The first scan line group (SG_{A}) and the second scan line group (SG_{B}) are used to form the first frame 221 and the second frame 222, respectively.

Although it is explained herein that the first and second scan line groups are set based on the reference scan line group, the present invention is certainly not limited thereto. It should be appreciated that at least two first scan line groups and at least two second scan line groups may be set based on the reference scan line group.

The controller 122 may be configured to perform control in such a manner that ultrasound signals are transmitted and received along the scan lines of the respective scan line groups at every frame change in order to form a plurality of frames. According to one embodiment of the present invention, the controller 122 may be configured to perform control to transmit and receive ultrasound signals along the scan lines (S_{A1} to S_{AN}) of the first scan line group (SG_{A}) and the scan lines (S_{B1} to S_{BN}) of the second scan line group (SG_{B}) in order to form the first frame 221 and the second frame 222, respectively. The controller 122 may control the formation of the frames of each scan line group, the storage of each frame and the spatial compound of each frame.

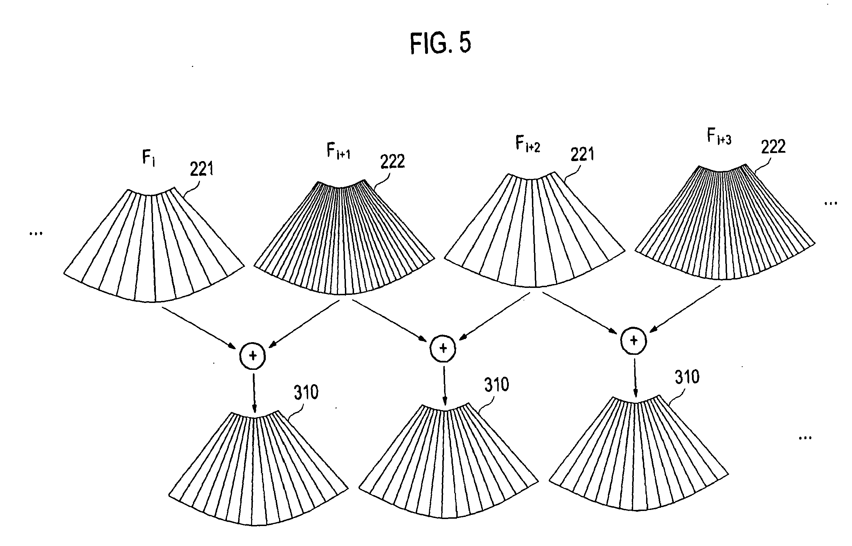

The beam former 130 delays and sums the ultrasound signals transmitted and received by a plurality of transducers along the scan lines of the respective scan line groups corresponding to the respective frames, thereby forming a plurality of frames. The operation of the beam former 130 is controlled by the controller 122. The frame forming unit 140 forms a frame corresponding to each scan line group based on the receive-focused signals, which are outputted from the beam former 130. The storage unit 150 stores in sequential manner frames of each scan line group, which is outputted from the frame forming unit 140. The compound image producing unit 160 extracts a plurality of frames from the storage unit 150 and forms a compound image by spatial compound of the extracted frames under the control of the controller 122.
According to one embodiment of the present invention, the compound image producing unit 160 extracts the first and second frames (221 and 222) from the storage unit 150, as shown in Figure 5. The compound image producing unit 160 forms the compound image 310 by spatial compound of the "i"th first frame (Fᵢ) and the "i+1"th second frame. Then, the compound image producing unit 160 forms the compound image 310 by spatial compound of the "i+1"th second frame (Fᵢ₊₁) and the "i+2"th first frame (Fᵢ₊₂). By doing so, the compound image producing unit 160 forms a plurality of compound images. In such a case, the compound image producing unit 160 may give different weights to the respective frames and compound the weighted frames. For example, the compound image producing unit 160 gives the first weight (e.g., 0.3) and the second weight (e.g., 0.7) to the first frame 221 and the second frame 222, respectively, and carries out spatial compound of the first and second frames. In such a case, the compound image is formed by spatial compound of two frames. However, it should be noted herein that the number of frames used for spatial compound is not limited to two. The compound image formed in the compound image producing unit 160 is displayed by the display 170.

## Claims

1. An ultrasound system (100) for forming an ultrasound image by using a plurality of frames, comprising a plurality of transducers (112) for transmitting ultrasound signals to an object and receiving the ultrasound signals reflected from the object to form a reception signal, a setting unit (121) for setting a plurality of scan line groups each having a scan line density based on a predetermined reference scan line group including a plurality of scan lines each having a different predetermined reference scan line density;
a controller (122) for controlling the transmission and reception of the ultrasound signals so as to form frame data corresponding to the respective scan line groups at every frame change;
a frame forming unit (140) for forming a plurality of frames corresponding to the respective scan line groups based on multiply formed frame data; and
a compound image forming unit (160) for forming a compound image by spatial compound of the plurality of frames,
**characterized in that**
the setting unlit (121) includes a scan line group setting unit for setting a first scan line group including a plurality of scan lines each having a lower scan line density than the reference scan line density and a second scan line group including a plurality of scan lines each having a higher scan line density than the reference scan line density.

2. The ultrasound system of Claim 1, wherein the controller (122) controls the reception and transmission of the ultrasound signals to alternately obtain the frame data corresponding to the first and second scan line groups at every frame changes.

3. The ultrasound system of Claim 1, wherein the compound image forming unit (160) includes:
a weight supplying unit for providing different weights to the frames corresponding to the respective scan line groups; and
a spatial compounding unit for forming a compound image by spatial compound of the weighted frames.

4. The ultrasound system of Claim 3, wherein the weight supplying unit provides the first and second weights to the frames corresponding to the first and second scan line groups, respectively, and wherein the first weight is less than the second weight.

5. The ultrasound system of Claim 1, further comprising:
a storage unit (150) for storing a plurality of frames.

6. A method of forming an ultrasound image by using a plurality of frames, comprising:
a) setting a plurality of scan line groups having a plurality of scan lines each having a scan line density based on a predetermined reference scan line group including a plurality of scan lines each having a different predetermined scan line density;
b) transmitting and receiving ultrasound signals to and from an object along the scan lines each of the scan line groups to form frame data;
c) forming frames corresponding to the respective scan line groups based on multiply formed frame data; and
d) forming a compound image by spatial compound of the frames corresponding to the respective scan line groups;
**characterized in that**
the step a) includes setting a first scan line group including a plurality of scan lines each having a lower scan line density than the reference scan line density and a second scan line group including a plurality of scan lines each having a higher scan line density than the reference scan line density.

7. The method of Claim 6, further comprising:
controlling the reception and transmission of the ultrasound signals to alternately obtain the frame data corresponding to the first and second scan line groups at every frame changes.

8. The method of Claim 6, further comprising:
providing different weights to the frames corresponding to the respective scan line groups; and
forming a compound image by spatial compound of the weighted frames.

9. The method of Claim 8, wherein the step of providing different weights provides the first and second weights to the frames corresponding to the first and second scan line groups, respectively.

10. The method of Claim 9, wherein the first weight is less than the second weight.

## Patentansprüche

1. Ultraschallsystem (100) zum Erzeugen eines Ultraschallbilds durch Verwendung einer Vielzahl von Frames bzw. Einzelbildern, mit einer Vielzahl von Transducern (112) zum Übertragen von Ultraschallsignalen zu einem Objekt und zum Empfangen der von dem Objekt reflektierten Signale, um ein Empfangssignal zu erzeugen, mit einer Einstelleinheit (121) zum Einstellen einer Vielzahl von Scanliniengruppen, welche jeweils eine Scanliniendichte aufweisen, die auf einer vorbestimmten Referenzscanliniengruppe einschließlich einer Vielzahl von Scanlinien basiert, die jede eine unterschiedliche vorbestimmte Referenzkennliniendichte aufweist; mit einer Steuereinheit (122) zum Steuern der Übertragung und des Empfangs der Ultraschallsignale, um Framedaten zu erzeugen, welche den entsprechenden Scanliniengruppen zu jedem Framewechsel entsprechen;
eine Frameerzeugungseinheit (140) zum Erzeugen einer Vielzahl von Frames, welche den entsprechenden Scanliniengruppen basierend auf mehrfach erzeugten Framedaten entsprechen; und
eine Verbundbild-Erzeugungseinheit (160) zum Erzeugen eines verbundenen Bilds bzw. Verbundbilds durch räumliche Verbindung der Vielzahl von Frames,
**dadurch gekennzeichnet, dass** die Einstelleinheit (121) eine Scanliniengruppen-Einstelleinheit zum Einstellen einer ersten Scanliniengruppe, welche eine Vielzahl von Scanlinien aufweist, die jede eine geringere Scanliniendichte als die Referenzscanliniendichte aufweisen, und einer zweiten Scanliniengruppe umfasst, welche eine Vielzahl von Scanlinien aufweist, die jede eine höhere Scanliniendichte aufweist als die Referenzscanliniendichte.

2. Ultraschallsystem nach Anspruch 1, wobei die Steuereinheit (122) den Empfang und die Übertragung der Ultraschallsignale steuert, um abwechselnd die Framedaten zu erlangen, welche den ersten und zweiten Scanliniengruppen bei jedem Framewechsel entsprechen.

3. Ultraschallsystem nach Anspruch 1, wobei die Verbundbild-Erzeugungseinheit (160) Folgendes aufweist:
eine Gewichtungs-Bereitstellungseinheit zum Bereitstellen von unterschiedlichen Gewichtungen für die Frames entsprechend der jeweiligen Scanliniengruppen; und
eine räumliche Verbindungseinheit zum Erzeugen eines Verbundbilds durch räumliche Verbindung der gewichteten Frames.

4. Ultraschallsystem nach Anspruch 3, wobei die Gewichtungs-Bereitstellungseinheit jeweils die ersten und zweiten Gewichtungen der Frames entsprechend der ersten und zweiten Scanliniengruppen zur Verfügung stellt, und wobei die erste Gewichtung geringer ist als die zweite Gewichtung.

5. Ultraschallsystem nach Anspruch 1, welches des weiteren Folgendes aufweist:
eine Speichereinheit (150) zum Speichern einer Vielzahl von Frames.

6. Verfahren zum Erzeugen eines Ultraschallbilds unter Verwendung einer Vielzahl von Frames bzw. Einzelbildern, welches Folgendes aufweist:
a) Einstellen einer Vielzahl von Scanliniengruppen, welche eine Vielzahl von Scanlinien aufweisen, welche jeweils eine Scanliniendichte basierend auf einer vorbestimmten Referenzscanliniengruppe einschließlich einer Vielzahl von Scanlinien aufweist, die jede eine unterschiedliche vorbestimmte Scanliniendichte aufweist;
b) Übertragen und Empfangen von Ultraschallsignalen zu und von einem Objekt entlang der Scanlinien von jeder der Scanliniengruppen, um Framedaten zu erzeugen;
c) Erzeugen von Frames entsprechend der jeweiligen Scanliniengruppen, basierend auf mehrfach erzeugten Framedaten; und
d) Erzeugen eines Verbundbilds durch räumliches Verbinden der Frames entsprechend der jeweiligen Scanliniengruppen;
**dadurch gekennzeichnet, dass** der Schritt a) das Einstellen einer ersten Scanliniengruppe, welche eine Vielzahl von Scanlinien aufweist, die jede eine geringere Scanliniendichte als die Referenzscanliniendichte aufweisen, und einer zweiten Scanliniengruppe umfasst, welche eine Vielzahl von Scanlinien aufweist, die jede eine höhere Scanliniendichte aufweist als die Referenzscanliniendichte.

7. Verfahren nach Anspruch 6, welches des weiteren Folgendes aufweist:
Steuern des Empfangs und der Übertragung der Ultraschallsignale, um abwechselnd die Framedaten zu erlangen, welche den ersten und zweiten Scanliniengruppen bei jedem Framewechsel entsprechen.

8. Verfahren nach Anspruch 6, welches des weiteren Folgendes aufweist:
Bereitstellen von unterschiedlichen Gewichtungen für die Frames entsprechend der jeweiligen Scanliniengruppen; und
Bilden eines Verbundbilds durch räumliche Verbindung der gewichteten Frames.

9. Verfahren nach Anspruch 8, wobei der Schritt des Bereitstellens unterschiedlicher Gewichtungen die ersten und zweiten Gewichtungen für die Frames jeweils entsprechend der ersten und zweiten Scanliniengruppen bereitstellt.

10. Verfahren nach Anspruch 9, wobei die erste Gewichtung niedriger ist als die zweite Gewichtung.

## Revendications

1. Système ultrasonore (100) destiné à former une image ultrasonore en utilisant une pluralité de trames, comprenant une pluralité de transducteurs (112) pour émettre des signaux ultrasonores vers un objet et recevoir les signaux ultrasonores réfléchis par l'objet pour former un signal de réception, une unité de paramétrage (121) pour paramétrer une pluralité de groupes de lignes de balayage ayant chacun une densité de lignes de balayage basée sur un groupe de lignes de balayage de référence prédéterminé qui comprend une pluralité de lignes de balayage dont chacune a une densité de ligne de balayage de référence prédéterminée différente,
un dispositif de commande (122) pour commander l'émission et la réception des signaux ultrasonores de manière à former des données de trames qui correspondent aux groupes de lignes de balayage respectifs à chaque changement de trame ;
une unité de formation de trames (140) destinée à former une pluralité de trames correspondant aux groupes de lignes de balayage respectifs sur la base de données de trames formées de façon multiple, et
une unité de formation d'images composées (160) pour former une image composée par composition spatiale de la pluralité de trames,
**caractérisé en ce que**
l'unité de paramétrage (121) comprend une unité de paramétrage de groupes de lignes de balayage pour paramétrer un premier groupe de lignes de balayage comprenant une pluralité de lignes de balayage ayant chacune une densité de ligne de balayage inférieure à la densité de ligne de balayage de référence et un second groupe de lignes de balayage comprenant une pluralité de lignes de balayage ayant chacune une densité de ligne de balayage supérieure à la densité de la ligne de balayage de référence.

2. Système ultrasonore de la revendication 1, dans lequel le dispositif de commande (122) commande la réception et l'émission des signaux ultrasonores pour obtenir alternativement les données de trames correspondant aux premier et second groupes de lignes de balayage à chaque changement de trame.

3. Système ultrasonore de la revendication 1, dans lequel l'unité de formation d'images composées (160) comprend :
une unité de fourniture de poids pour appliquer différents poids aux trames correspondant aux groupes de lignes de balayage respectifs, et
une unité de composition spatiale pour former une image composée par composition spatiale des trames pondérées.

4. Système ultrasonore de la revendication 3, dans lequel l'unité de fourniture de poids applique les premier et second poids aux trames correspondant respectivement au premier et au second groupe de lignes de balayage, et dans lequel le premier poids est inférieur au second poids.

5. Système ultrasonore de la revendication 1, comprenant en outre :
une unité de stockage (150) pour stocker une pluralité de trames.

6. Procédé pour former une image ultrasonore en utilisant une pluralité de trames, comprenant :
a) le paramétrage d'une pluralité de groupes de lignes de balayage dont chacun possède une densité de ligne de balayage basée sur un groupe de lignes de balayage de référence prédéterminé comprenant une pluralité de lignes de balayage, chacune ayant une densité de ligne de balayage prédéterminée différente,
b) l'émission et la réception de signaux ultrasonores vers et d'un objet le long des lignes de balayage de chacun des groupes de lignes de balayage pour former des données de trames,
c) la formation de trames correspondant aux groupes de lignes de balayage respectives sur la base de données de trames formées de façon multiple, et
d) la formation d'une image composée par composition spatiale des trames correspondant aux groupes de lignes de balayage respectifs,
**caractérisé en ce que**
l'étape a) comprend le paramétrage d'un premier groupe de lignes de balayage comprenant une pluralité de lignes de balayage ayant chacune une densité de ligne de balayage inférieure à la densité de ligne de balayage de référence et d'un second groupe de lignes de balayage comprenant une pluralité de lignes de balayage ayant chacune une densité de ligne de balayage supérieure à la densité de ligne de balayage de référence.

7. Procédé de la revendication 6, comprenant en outre :
la commande de la réception et de l'émission des signaux ultrasonores pour obtenir alternativement les données de trames correspondant au premier et au second groupe de lignes de balayage à chaque changement de trame.

8. Procédé de la revendication 6, comprenant en outre :
l'application de différents poids aux trames correspondant aux groupes de lignes de balayage respectifs, et
la formation d'une image composée par composition spatiale des trames pondérées.

9. Procédé de la revendication 8, dans lequel l'étape d'application des différents poids applique les premier et second poids aux trames correspondant respectivement au premier et au second groupe de lignes de balayage.

10. Procédé de la revendication 9, dans lequel le premier poids est inférieur au second poids.
